# EUROPÄISCHE PATENTSCHRIFT

(11) **EP 1 462 161 B1**
(45) Veröffentlichungstag und Bekanntmachung des Hinweises auf die Patenterteilung: **30.06.2010**
(21) Anmeldenummer: 04007199.5
(22) Anmeldetag: 25.03.2004
(51) Int. Cl.: B01J 19/02, C07C 2/00, B01J 19/00, B01J 12/00

(54) **Reaktor für Hochtemperaturreaktionen, seine Herstellung und Verwendung**
Reactor for high-temperature reactions, its manufacture and use
Dispositif pour la réalisation de réactions à haute température ainsi que sa fabrication et son utilisation

(30) Priorität: 26.03.2003 DE 10313529
(43) Veröffentlichungstag der Anmeldung: 29.09.2004
(73) Patentinhaber: BASF SE, 67056 Ludwigshafen (DE)
(72) Erfinder: Bartenbach, Bernd, Dr., 67117 Limburgerhof (DE)

(56) Entgegenhaltungen:
- WO-A-01/37984
- GB-A- 2 121 313
- US-A- 2 765 358
- US-A- 3 287 434
- US-A- 3 438 741
- US-A- 5 407 455
- US-A- 5 932 182
- US-B1- 6 258 330

## Beschreibung

Die Erfindung betrifft einen Reaktor für Hochtemperaturreaktionen sowie eine Verwendung des Reaktors zur Herstellung von Acetylen.

Bei herkömmlichen Verfahren, die bei hohen Temperaturen ablaufen, werden häufig Brenner mit metallischen Wänden eingesetzt, wobei die Wände meist durch ein Kühlmedium gekühlt werden. Eine derartige Hochtemperaturreaktion ist die Herstellung von Acetylen und Synthesegas durch partielle Oxidation von Kohlenwasserstoffen, die beispielsweise in DE-A 44 22 815 beschrieben ist.

Danach werden die Ausgangsstoffe Erdgas und Sauerstoff üblicherweise möglichst auf bis zu 700°C getrennt vorgeheizt, in einer Mischzone intensiv vermischt und nach Durchströmen eines Brennerblocks zur Reaktion gebracht. Der Brennerblock besteht aus einer bestimmten Anzahl von Kanälen, in denen die Geschwindigkeit der Reaktion wegen der zündfähigen Sauerstoff/Erdgas-Mischung höher ist als die Flammengeschwindigkeit, um ein Durchschlagen der Flamme in den Mischraum zu verhindern. Der sich dem Brennerblock anschließende Reaktionsraum ist so bemessen, dass bei einer bestimmten Einsatzstoffmenge die Verweilzeit des acetylenhaltigen Reaktionsgases, des sogenannten Spaltgases, nur wenige Millisekunden beträgt. Nach dieser Zeit, innerhalb der sich die dem Temperaturniveau von 1500 bis 2000°C entsprechenden Gleichgewichte nicht einstellen können, werden die Reaktionsprodukte möglichst augenblicklich auf unter 300°C mit Wasser oder vorzugsweise Rückstandsöl abgeschreckt, damit das gebildete Acetylen nicht in Russ und Wasserstoff zerfällt. Die Zerfallsreaktion des Acetylens lässt sich jedoch nicht vollständig unterdrücken, mit der Folge, dass sich elementarer Kohlenstoff bildet, der sich als Russ und Koksanbackungen an den Wänden des Reaktionsraumes absetzt.

In einem Bericht der Chemie Ingenieur Technik (CIT, 26 (1954) 5, S. 251) wird von einer Brennerauskleidung aus Schamotte für einen Brenner im Kleinstmaßstab für die Acetylenherstellung nach dem Sachsse-Bartholomé-Acetylenverfahren berichtet. Diese Bauform wurde aber zugunsten gekühlter Metalloberflächen fallengelassen, da aufgrund der geringen Standzeiten der keramischen Beschichtung, deren Anwendungsgrenztemperatur unterhalb der Prozesstemperatur lag, keine Vorteile erkennbar waren. Konstruktive Lösungen für größere Maßstäbe in der Größenordnung von 25 Tagestonnen Acetylen, wie sie zum Beispiel heute eingesetzt werden, gerade bei keramischen Beschichtungen unter diesen thermischen Belastungen, waren nicht vorhanden.

Bei partiell oxidativen und pyrolytischen Verfahren, denen auch die Herstellung von Acetylen zuzuordnen ist, entsteht eine erhebliche Menge an Ruß bei den Spaltvorgängen der eingesetzten Kohlenwasserstoffe. Der Ruß setzt sich, besonders während der Bildungsphase, wegen seiner hohen Oberflächenaktivität durch thermophoretische Vorgänge und Kondensationsvorgänge bevorzugt auf kalten Oberflächen ab. Dieser Effekt ist besonders stark im Bereich von Rückströmzonen, wie sie zum Beispiel an den Zwickelgebieten der Brennerbohrungen auftreten. Die im Verlauf der Reaktion zunehmende Dicke der Rußschicht führt zu einer sukzessiven Erhöhung der Isolierwirkung gegen die gekühlte Metallwand. Aufgrund dieser Isolierwirkung werden die Rußschichten thermischen Crack- und Verkokungsvorgängen unterworfen. Hierdurch wandeln sich die Rußschichten in harte Koksanbackungen um. Die Koksanbackungen wachsen aufgrund der heißen, reaktiven Oberfläche durch weitere Russablagerung weiter an. Das führt dazu, dass die Koksablagerungen mechanisch abgereinigt werden müssen. Früher geschah dies von Hand, heute werden dazu aufwendige Stocherroboter eingesetzt. Der durch die mechanische Reinigung abgestocherte Koks weist eine grobe Struktur auf und ist sehr hart. Der Koks wird zusammen mit dem Gasphasenruß in das unverdampfte überschüssige Quenchmedium aufgenommen und mit diesem ausgetragen. Aufgrund der groben Struktur wirkt der abgestocherte Koks abrasiv an nachfolgenden Anlagenteilen.

Aus US 3,287,434 ist ein Verfahren zur Herstellung von Acetylen durch Partialoxidation von Kohlenwasserstoffen mit einem Sauerstoff enthaltenden Strom bekannt, wobei das Reaktionsgemisch in einem Brennerblock auf eine Temperatur oberhalb der Zündtemperatur vorgeheizt wird und das vorgeheizte Reaktionsgemisch in eine Verbrennungszone mit einer so hohen Geschwindigkeit geleitet wird, dass die Flammeninduktionsperiode auf dem Weg in eine Verbrennungszone mit einer so hohen geschwindigkeit geleitet wird, dass die Flammeninduktionsperiode auf dem Wege durch den Brennerblock unterschritten wird. Der Brennerblock ist aus nicht-metallischen, hochtemperaturbeständigen Materialien, beispielsweise Aluminiumoxid, gebildet.

Dokument US 3,438,741 beschreibt einen weiteren Apparat zur Herstellung von Acetylen durch Partialoxidation von Kohlenwasserstoffen mit einer Mischkammer für das Reaktionsgemisch, einem Gasverteiler und einer Reaktionskammer. Der Gasverteiler ist aus keramischem Material, beispielsweise Aluminiumoxid, gebildet, worauf metallische Oberflächen aufgebracht sind.

Es war daher Aufgabe der Erfindung, einen Reaktor für eine Hochtemperaturreaktion mit kurzer Verweilzeit und mit anschließender schneller Abkühlung des Reaktionsgemisches in einem Quenchbereich zur Verfügung zu stellen, der die oben genannten Nachteile nicht aufweist.

Die Aufgabe wird gelöst durch einen Reaktor mit Zuführung eines Reaktionsgemisches über Kanäle eines Brennerblocks in einem Reaktionsraum, wobei im Reaktionsraum eine Hochtemperaturreaktion mit kurzer Verweilzeit stattfindet und das Reaktionsgemisch anschließend in einem Quenchbereich schnell abgekühlt wird, wobei alle den Reaktionsraum begrenzenden Oberflächen aus einer bei Reaktionstemperatur beständigen Feuerfestkeramik mit einem Aluminiumoxidanteil von mindestens 80% ausgebildet sind. Die Erfindung ist grundsätzlich nicht eingeschränkt bezüglich des Werkstoffs für den Grundkörper, auf den die Feuerfestkeramik aufzubringen ist. Hierfür werden metallische Werkstoffe eingesetzt.

Als Hochtemperaturreaktionen werden in der Regel Reaktionen bezeichnet, die bei einer Temperatur oberhalb von 800°C, insbesondere aber 1000°C stattfinden.

Als kurz werden vorliegend Verweilzeiten bezeichnet, die im Millisekunden-Bereich liegen, insbesondere im Bereich von etwa 1 bis 100 ms.

Für eine schnelle Abkühlung im Sinne der vorliegenden Erfindung wird eine Abkühlung in einem mit der Verweilzeit der Hochtemperaturreaktion vergleichbaren Zeitintervall verstanden, das heißt ein Zeitintervall im Millisekunden-Bereich, bevorzugt im Bereich von etwa 1 bis 50 ms.

Um eine Russabscheidung und damit eine Koksbildung weitgehend oder vollständig zu verhindern, werden in der hier dargestellten erfindungsgemäßen Lösung die Wände des Reaktionsraumes mit einer Feuerfestkeramik ausgekleidet. Eine Beständigkeit der Keramik gegen die bei den Hochtemperaturreaktionen auftretenden Temperaturen von über 1650°C wird durch einen Aluminiumoxidanteil von mindestens 80%, bevorzugt von mindestens 95 Gew.-% und besonders bevorzugt von mindestens 96 Gew.-%, erreicht.

Die Auskleidung des Reaktionsraumes erfolgt in einer ersten Ausführungsvariante durch Ausmauern mit der Feuerfestkeramik in Form von Steinen oder Blöcken.

In einer zweiten Ausführungsvariante wird die Feuerfestkeramik in Form einer Gieß- oder Stampfmasse in den Reaktionsraum eingebracht und dort anschließend verdichtet, getrocknet und gebrannt. In einer bevorzugten Ausführungsform wird die als Gieß- oder Stampfmasse in den Reaktionsraum eingebrachte Feuerfestkeramik durch die Hochtemperaturreaktion gebrannt.

Die Feuerfestkeramik, mit der der Reaktionsraum ausgekleidet ist, weist vorteilhaft eine Dicke im Bereich von 7 bis 30 cm, bevorzugt eine Dicke im Bereich von 8 bis 10 cm, auf. Zusätzlich kann eine Hinterisolierung aus einer Keramik mit besonders guten wärmeisolierenden Eigenschaften erfolgen.

Ein Vorteil des erfindungsgemäß ausgebildeten Reaktionsraumes ist, dass die Auskleidung thermisch isolierend wirkt. Aus diesem Grund braucht die Wand des Reaktionsraumes nicht mehr zwingend gekühlt zu werden, was zu einer Einsparung an Kühlmedium und Konstruktionsaufwand für die Kühlmedium-Verteilung führt.

Ein weiterer Vorteil der erfindungsgemäßen Lösung ist, dass durch ausreichend gute Feuerfestisolierung selbst unter extremen Bedingungen, d.h. bei sehr hohen Temperaturen und starker Russbildung die Russabscheidung und damit die Bildung von Koks verhindert werden kann. Hierdurch lässt sich die mechanische Stochereinrichtung und deren aufwändige Wartung einsparen. Zudem entfallen durch die Einsparung der mechanischen Stochereinrichtung durch Stocherfehler ausgelöste Betriebsunterbrechungen. Schließlich wird die Materialbelastung durch die erosive Wirkung des abgestocherten Kokses auf die nachgeschalteten Anlageteile, wie Pumpen, Wärmeübertrager und Rohrleitungen, drastisch reduziert.

Besonders bedeutsam ist die Vermeidung des abgestocherten Kokses, wenn das bei der Reaktion entstehende Spaltgas aus dem Brenner in einem Wärmeübertrager zur Abhitzenutung ganz oder teilweise abgekühlt werden soll, wie in DE-A 199 14 226 beschrieben.

Im Folgenden wird die Erfindung anhand einer Zeichnung und eines Ausführungsbeispiels näher erläutert.

Es zeigt:
- Figur 1: einen Ausschnitt aus einem Reaktor, der einen Brennerblock und eine Ausführungsform eines erfindungsgemäß gestalteten Reaktionsraumes umfasst.
- Figur 2: einen Reaktor zur Acetylenherstellung nach dem Sachsse-Bartholomé-Verfahren gemäß dem Stand der Technik,

In den Figuren bezeichnen gleiche Bezugszeichen gleiche oder entsprechende Merkmale.

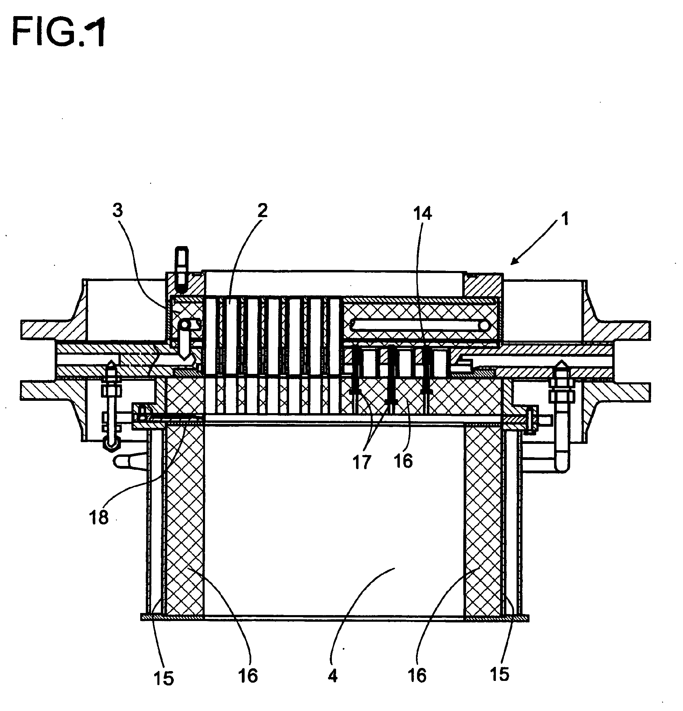

Figur 1 zeigt einen Ausschnitt aus einem erfindungsgemäß ausgebildeten Reaktor 1 zur Acetylenherstellung mit Brennerblock 3 und Reaktionsraum 4. Im Brennerblock 3 befinden sich neben den Kanälen 2 zur Zufuhr des Reaktionsgemisches Zusatzkanäle 14, über die zusätzlicher Sauerstoff oder Reaktionshilfsstoffe in den Reaktionsraum 4 gelangen können. Der hier dargestellte erfindungsgemäß ausgebildete Reaktionsraum 4 weist Seitenwände 15 auf, die mit einer Feuerfestkeramik 16 ausgekleidet sind. Neben den Seitenwänden 15 ist auch die Wand des Reaktionsraumes 4, die durch den Brennerblock 3 begrenzt wird, mit Feuerfestkeramik 16 ausgekleidet. Die Kanäle 2 zur Zufuhr des Reaktionsgemisches und die Zusatzkanäle 14 für zusätzlichen Sauerstoff oder Reaktionshilfsstoffe werden durch die Feuerfestkeramik 16 hindurch verlängert.

Um eine Rissbildung aufgrund unterschiedlicher thermischer Ausdehnung bei hohen Temperaturen in der Feuerfestkeramik zu verhindern, werden vorzugsweise in der metallischen Grundkonstruktion des Reaktors 1 Anker und Dehnfugen integriert.

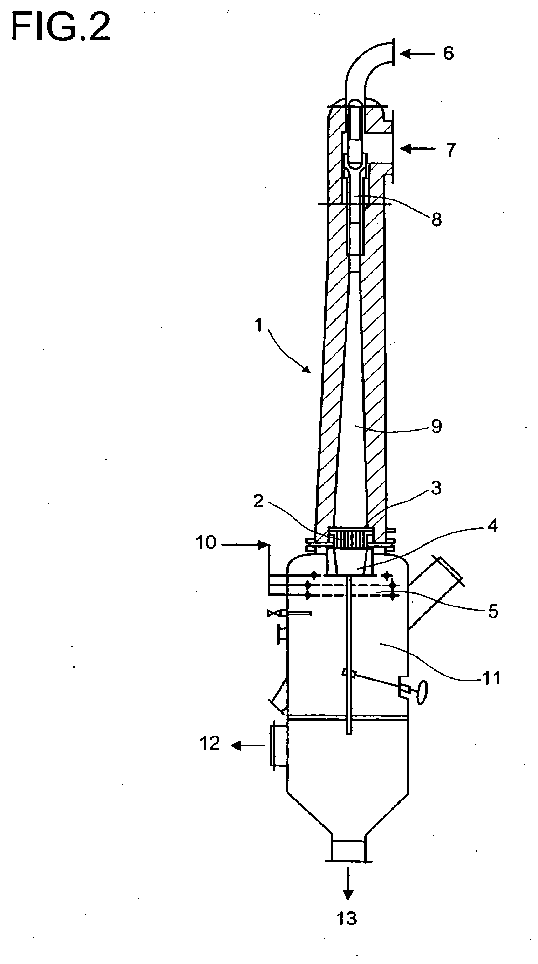

Demgegenüber ist in Figur 2 ein Reaktor 1 zur Acetylenherstellung nach dem Stand der Technik dargestellt. Dem Reaktor wird über eine Sauerstoffzufuhr 6 Sauerstoff oder ein sauerstoffhaltiges Gas und über eine Kohlenwasserstoffzufuhr 7 ein Kohlenwasserstoff oder ein Kohlenwasserstoffgemisch zugeführt. Der Sauerstoff oder das sauerstoffhaltige Gas und der Kohlenwasserstoff oder das Kohlenwasserstoffgemisch werden in einer Mischzone 8 vermischt und über einen Diffusor 9 einem mit Kanälen 2 versehenen Brennerblock 3 zugeführt. Über die Kanäle 2 des Brennerblocks 3 gelangt das Reaktionsgemisch in einen Reaktionsraum 4. Im Reaktionsraum 4 wird das Gemisch in einer Flamme partiell zu Acetylen und Synthesegas oxidiert. Um Folgereaktionen zu vermeiden, wird das Gemisch direkt im Anschluss in einem Quenchbereich 5 schnell abgekühlt. Zur schnellen Abkühlung wird dem Quenchbereich 5 über eine Kühlmittelleitung 10 Kühlmittel zugeführt und direkt in das Reaktionsgemisch eingemischt. Die Abkühlung erfolgt im Quenchbehälter 11 unter teilweiser Verdampfung des Kühlmittels. Im Anschluss an die Abkühlung wird das Spaltgas über den Spaltgasabzug 12 und das Kühlmittel über den Kühlmittelauslass 13 aus dem Reaktor abgeführt.

### Ausführungsbeispiel

Auf der Basis eines konventionellen Reaktors zur Acetylenherstellung wurde die Wirksamkeit der keramischen Auskleidung hinsichtlich der Russabscheidung untersucht. Bei dem Reaktor mit einem Reaktionsraumdurchmesser von 533 mm gemäß Figur 2 zur Acetylenherstellung nach dem Sachsse-Bartholomé-Verfahren wurde die metallische Grundkonstruktion geringfügig modifiziert und der Reaktionsraum mit einer Feuerfestkeramik mit einer Dicke von 8 cm ausgekleidet. Beim Betrieb des Reaktors zeigte sich, dass eine Russabscheidung und damit Koksbildung an den Oberflächen des Reaktionsraumes nahezu vollständig vermieden werden konnte.

Zudem zeigte sich keine Schädigung an der Feuerfestkeramik im Reaktionsraum. Dies war vor allem insofern erstaunlich, als die Anwendungs-Grenztemperaturen der im allgemein verfügbaren und zur Auskleidung des Reaktionsraumes geeigneten Feuerfestkeramiken nur geringfügig über den Prozesstemperaturen der partiell oxidativen Acetylenherstellung liegen.

### Bezugszeichenliste

- 1: Reaktor
- 2: Kanäle
- 3: Brennerblock
- 4: Reaktionsraum
- 5: Quenchbereich
- 6: Sauerstoffzufuhr
- 7: Kohlenwasserstoffzufuhr
- 8: Mischzone
- 9: Diffusor
- 10: Kühlmittelleitung
- 11: Quenchbehälter
- 12: Spaltgasabzug
- 13: Kühlmittelauslass
- 14: Zusatzkanäle
- 15: Seitenwand
- 16: Feuerfestkeramik
- 17: Anker
- 18: Dehnfugen

## Patentansprüche

1. Reaktor (1) zur Durchführung einer Hochtemperaturreaktion mit kurzer Verweilzeit, mit einem Brennerblock (3) mit Kanälen (2) für die Zuführung eines Reaktionsgemisches, mit einem Reaktionsraum (4), in dem die Hochtemperaturreaktion mit kurzer Verweilzeit stattfindet, und an den sich ein Quenchbereich (5) zur schnellen Abkühlung des Reaktionsgemisches anschließt, wobei der Reaktor aus einem Grundkörper aus metallischen Werkstoffen gebildet ist, **dadurch gekennzeichnet, dass** alle den Reaktionsraum (4) begrenzenden Oberflächen aus einer bei Reaktionstemperatur beständigen Feuerfestkeramik mit einem Aluminiumoxid-Anteil von mindestens 80 Gew.-% ausgebildet sind.

2. Reaktor (1) nach Anspruch 1, **dadurch gekennzeichnet, dass** der Aluminiumoxid-Anteil der Feuerfestkeramik mindestens 95 Gew.-% beträgt.

3. Reaktor (1) nach Anspruch 2, **dadurch gekennzeichnet, dass** der Aluminiumoxid-Anteil der Feuerfestkeramik mindestens 96 Gew.-% beträgt.

4. Reaktor (1) nach einem der Ansprüche 1 bis 3, **dadurch gekennzeichnet, dass** die Feuerfestkeramik eine Dicke im Bereich von 7 bis 30 cm, bevorzugt eine Dicke im Bereich von 8 bis 10 cm, aufweist.

5. Verfahren zur Herstellung des Reaktors (1) nach einem der Ansprüche 1 bis 4, **dadurch gekennzeichnet, dass** die Feuerfestkeramik in Form von Steinen oder Blöcken oder als Gieß- oder Stampfmasse in den Reaktionsraum eingebracht und anschließend verdichtet, getrocknet und gebrannt ist.

6. Verfahren nach Anspruch 5, **dadurch gekennzeichnet, dass** die Gieß- oder Stampfmasse durch die Hochtemperaturreaktion gebrannt ist.

7. Verwendung des Reaktors nach einem der Ansprüche 1 bis 4 zur Herstellung von Acetylen durch partielle Oxidation von Kohlenwasserstoffen mit Sauerstoff.

## Claims

1. A reactor (1) for carrying out a high-temperature reaction having a short residence time, having a burner block (3) having channels (2) for supply of a reaction mixture, having a reaction chamber (4) in which the high-temperature reaction having a short residence time takes place and which is followed by a quench area (5) for rapidly cooling the reaction mixture, the reactor being formed from a basic structure composed of metal materials, wherein all surfaces delineating the reaction chamber (4) are formed from a fire-resistant ceramic having an alumina content of at least 80% by weight, which is stable at reaction temperature.

2. The reactor (1) according to claim 1, wherein the alumina content of the fire-resistant ceramic is at least 95% by weight.

3. The reactor (1) according to claim 2, wherein the alumina content of the fire-resistant ceramic is at least 96% by weight.

4. The reactor (1) according to one of claims 1 to 3, wherein the fire-resistant ceramic has a thickness in the range from 7 to 30 cm, preferably a thickness in the range from 8 to 10 cm.

5. A process for producing the reactor (1) according to one of claims 1 to 4, which comprises the fire-resistant ceramic being introduced into the reaction chamber in the form of stones or blocks or as a cast or tamped mass and then compressed, dried and calcined.

6. The process according to claim 5, wherein the cast or tamped mass is calcined by means of the high-temperature reaction.

7. The use of the reactor according to one of claims 1 to 4 for the preparation of acetylene by partial oxidation of hydrocarbons with oxygen.

## Revendications

1. Réacteur (1) pour la réalisation d'une réaction à température élevée et temps de séjour court, comportant un bloc brûleur (3) muni de canaux (2) pour l'introduction d'un mélange réactionnel, comportant une chambre de réaction (4) dans laquelle la réaction à température élevée et temps de séjour court a lieu, et à laquelle une zone de refroidissement (5) pour le refroidissement rapide du mélange réactionnel est connectée, le réacteur étant formé à partir d'un corps de base en matériaux métalliques, **caractérisé en ce que** toutes les surfaces qui délimitent la chambre de réaction (4) sont formées à partir d'une céramique réfractaire résistante à la température de réaction qui contient une proportion d'oxyde d'aluminium d'au moins 80 % en poids.

2. Réacteur (1) selon la revendication 1, **caractérisé en ce que** la proportion d'oxyde d'aluminium de la céramique réfractaire est d'au moins 95 % en poids.

3. Réacteur (1) selon la revendication 2, **caractérisé en ce que** la proportion d'oxyde d'aluminium de la céramique réfractaire est d'au moins 96 % en poids.

4. Réacteur (1) selon l'une quelconque des revendications 1 à 3, **caractérisé en ce que** la céramique réfractaire présente une épaisseur dans la plage allant de 7 à 30 cm, de préférence une épaisseur dans la plage allant de 8 à 10 cm.

5. Procédé de fabrication du réacteur (1) selon l'une quelconque des revendications 1 à 4, **caractérisé en ce que** la céramique réfractaire est introduite dans la chambre de réaction sous la forme de pierres ou de blocs ou sous la forme d'une masse de coulage ou de damage, puis comprimée, séchée et brûlée.

6. Procédé selon la revendication 5, **caractérisé en ce que** la masse de coulage ou de damage est brûlée par la réaction à température élevée.

7. Utilisation du réacteur selon l'une quelconque des revendications 1 à 4 pour la fabrication d'acétylène par oxydation partielle d'hydrocarbures avec de l'oxygène.
